# EUROPEAN PATENT APPLICATION

(11) **EP 3 702 779 A1**
(43) Date of publication of application: **02.09.2020**
(21) Application number: 18869728.8
(22) Date of filing: 18.10.2018
(51) Int. Cl.: G01N 33/48, G01N 21/64, G06T 7/571

(54) **IMAGE PROCESSING DEVICE, IN-FOCUS POSITION SPECIFYING METHOD, AND IN-FOCUS POSITION SPECIFYING PROGRAM**

(30) Priority: 26.10.2017 JP 2017206759
(71) Applicant: Konica Minolta, Inc., Tokyo 100-7015 (JP)
(72) Inventor: KEMMOCHI, Hiroaki, Tokyo 100-7015 (JP)
(74) Representative: Henkel & Partner mbB
(86) International application number: PCT/JP2018/038831
(87) International publication number: WO 2019/082788

(57) **Abstract**

The present invention addresses the problem of providing: an image processing device, an in-focus position specifying method, and an in-focus position specifying program capable of specifying an in-focus position from a fluorescence image and allowing an expression level of a biological material over the entirety of a tissue specimen to be quantitatively analyzed. The present invention is provided with: an input means for inputting a morphology image representing the morphology of cells in a tissue specimen in which a biological material is stained by fluorescent nanoparticles and fluorescence images which represent the same range as that of the morphology image, which represent the expression of the biological material, and in which the focal planes are changed at a predetermined interval in the height direction of the tissue specimen; a first extraction means for extracting cell areas from the morphology image or the fluorescence images; an in-focus plane specifying means for specifying, as an in-focus plane, the focal plane being most focused for each of the cell areas; a coordinate specifying means for specifying coordinates in the in-focus plane of the cell area; a storage means for storing the in-focus plane and the coordinates; a second extraction means for extracting a fluorescent bright point area from a fluorescence image in a focal plane corresponding to the in-focus plane; and a calculation means for calculating the number of fluorescent nanoparticles or a brightness value in the fluorescent bright spot area.

## Description

### TECHNICAL FIELD

The present invention relates to an image processing device, an in-focus position specifying method, and an in-focus position specifying program.

### BACKGROUND ART

There have been conventionally known immunostaining methods of detecting an antigen in a tissue sample as cancer diagnostics. Among the immunostaining methods, the fluorescent antibody method has been more commonly used in recent years instead of the enzyme antibody method. The "fluorescent antibody method" is a technique of beforehand labeling an antibody with a fluorescent substance, staining a tissue sample (antigen-antibody reaction), and then irradiating the tissue sample with excitation light to emit fluorescence, so that this emission is observed with a fluorescent microscope. Patent Document 1 discloses an example of using the fluorescent antibody method. In particular, Patent Document 1 proposes a method of evaluating the expression level of biological substance by observing the fluorescent substance bonded to the biological substance with the fluorescent microscope and measuring the number of fluorescent bright spots and the fluorescent intensity.

In order to measure the above number of fluorescent bright spots and fluorescent intensity, an optical system having high magnification and numerical aperture (NA) is required. The adjustment of in-focus position is important since the depth of focus becomes narrow when the magnification and/or the numerical aperture of the optical system is increased. The "in focus" indicates being focused, and when the position is out of focus, there is a possibility that the fluorescent bright spot may be missed in the microscopic image (fluorescent image) including the fluorescent bright spot.

As for this point, the specifying method of in-focus position of the fluorescent image is disclosed in Patent Document 2.

In Patent Document 2, the imaging range is divided into multiple ranges in the height direction (Z direction) of the tissue sample, for each divided imaging range, the stage is moved at a constant speed upward in the Z direction to capture the fluorescent image. Thereafter, the frequency analysis is executed to the multiple fluorescent images and the fluorescent image having the highest maximum frequency component is selected. The image of trace of the fluorescent marker is analyzed for the selected fluorescent image, and on the basis of the analysis result, the multiple fluorescent marker distribution information is calculated. Furthermore, data of histogram form is generated from the fluorescent marker distribution information, and the in-focus position is specified from the histogram.

### PRIOR ART DOCUMENT

### PATENT DOCUMENTS

Patent Document 1: Japanese Patent Application Laid Open Publication No. 2013-57631
Patent Document 2: Japanese Patent Application Laid Open Publication No. 2013-114042

### DISCLOSURE OF THE INVENTION

### PROBLEMS TO BE SOLVED BY THE INVENTION

In the conventional staining using the fluorescent dye, the fluorescent dye penetrates not only the cell surface but inside the cell, and thus, the in-focus position can be specified relatively easily by varying the position of focal point. However, the fluorescent labeling by the fluorescent dye is poor in luminance stability and color fading of the fluorescent dye itself easily occurs, which is not suitable for quantitative analysis.

On the other hand, fluorescent labels of high luminance such as quantum dots and fluorescent substance-containing nanoparticles containing the fluorescent dye enable measurement of the number of fluorescent bright spots and the fluorescent intensity using the fluorescent microscope, which is suitable for quantitative analysis. However, since these fluorescent labels are specifically adsorbed to the cell surface, the fluorescent labels are adsorbed along unevenness of the surface of the tissue sample, while the cells are distributed also in the height direction. Accordingly, when the fluorescent image is captured on a focal plane, both of cells which are in focus and cells which are not in focus exist. In the in-focus position specifying method described in Patent Document 2, though imaging is performed while changing the focal plane in the height direction of the tissue sample, only the fluorescent information in a same focal plane is obtained from a single fluorescent image. Thus, it is difficult to quantify the total number of the fluorescent bright spots adsorbed on the surface of tissue sample with high accuracy and high reproducibility. When the expression amount of biological substance is small or the biological substance is not expressed, it is difficult to determine the focal plane from the fluorescent information, which makes the quantitative analysis difficult.

The present invention has been made in consideration of the above problems, and an object of the present invention is to provide an image processing device, an in-focus position specifying method, and an in-focus position specifying program that specify the in-focus position from the fluorescent image and enable quantitative analysis of the expression amount of biological substance of the entire tissue sample.

### MEANS FOR SOLVING THE PROBLEM

In order to achieve the above object, the image processing device according to claim 1 is an image processing device including: an input means to input a morphological image and a plurality of fluorescent images, the morphological image representing a morphology of a cell in a tissue sample in which a biological substance is stained with a fluorescent nanoparticle, the biological substance being a single type of biological substance or a plurality of types of biological substances, and the plurality of fluorescent images having focal planes which are different at a predetermined interval in a height direction of the tissue sample in a same range as a range of the morphological image and representing expression of the biological substance in the tissue sample with a fluorescent bright spot; a first extraction means that extracts a cell region from the morphological image or a fluorescent image among the fluorescent images; an in-focus plane specifying means that specifies a focal plane which is most in focus as an in-focus plane for each of the cell region extracted by the first extraction means; a coordinate specifying means that specifies a coordinate in the in-focus plane of the cell region for each of the cell region extracted by the first extraction means; a storage means that stores the in-focus plane and the coordinate for each of the cell region extracted by the first extraction means; a second extraction means that extracts a fluorescent bright spot region from a fluorescent image among the fluorescent images which is in a focal plane, among the focal planes, corresponding to the in-focus plane for each of the cell region extracted by the first extraction means; and a calculation means that calculates a luminance value or a number of the fluorescent nanoparticle in the fluorescent bright spot region.

The invention according to claim 2 is the image processing device according to claim 1, wherein the morphological image is a plurality of morphological images having focal planes which are different at a predetermined interval in the height direction of the tissue sample and representing the morphology of the cell, and the fluorescent images are a plurality of fluorescent images representing, with the fluorescent bright spot, the expression of the biological substance in focal planes corresponding to the focal planes of the respective morphological images in same ranges as ranges of the respective morphological images.

The invention according to claim 3 is the image processing device according to claim 1 or 2, wherein the first extraction means extracts the cell region from the morphological image by recognizing a cell nucleus.

The invention according to claim 4 is the image processing device according to any one of claims 1 to 3, wherein the calculation means calculates the luminance value or a number of a fluorescent dye accumulated particle for each of the cell region extracted by the first extraction means.

The invention according to claim 5 is the image processing device according to any one of claims 1 to 4, further including an image compositing means that generates one composite image by extracting, for each of the cell region extracted by the first extraction means, a partial image of a part corresponding to the cell region from the fluorescent image in the focal plane corresponding to the in-focus plane based on the coordinate and compositing all of the extracted partial image of each of the cell region.

The in-focus position specifying method according to claim 6 is an in-focus position specifying method including: an input step that is inputting a morphological image and a plurality of fluorescent images, the morphological image representing a morphology of a cell in a tissue sample in which a biological substance is stained with a fluorescent nanoparticle, the biological substance being a single type of biological substance or a plurality of types of biological substances, and the plurality of fluorescent images having focal planes which are different at a predetermined interval in a height direction of the tissue sample in a same range as a range of the morphological image and representing expression of the biological substance in the tissue sample with a fluorescent bright spot; a first extraction step that is extracting a cell region from the morphological image or a fluorescent image among the fluorescent images; an in-focus plane specifying step that is specifying a focal plane which is most in focus as an in-focus plane for each of the cell region extracted by the first extraction step; a coordinate specifying step that is specifying a coordinate in the in-focus plane of the cell region for each of the cell region extracted by the first extraction step; a storage step that is storing the in-focus plane and the coordinate for each of the cell region extracted by the first extraction step; a second extraction step that is extracting a fluorescent bright spot region from a fluorescent image among the fluorescent images which is in a focal plane, among the focal planes, corresponding to the in-focus plane for each of the cell region extracted by the first extraction step; and a calculation step that is calculating a luminance value or a number of the fluorescent nanoparticle in the fluorescent bright spot region.

The in-focus position specifying program according to claim 7 is an in-focus position specifying program of a fluorescent image, the program causing a computer to function as: an input means to input a morphological image and a plurality of fluorescent images, the morphological image representing a morphology of a cell in a tissue sample in which a biological substance is stained with a fluorescent nanoparticle, the biological substance being a single type of biological substance or a plurality of types of biological substances, and the plurality of fluorescent images having focal planes which are different at a predetermined interval in a height direction of the tissue sample in a same range as a range of the morphological image and representing expression of the biological substance in the tissue sample with a fluorescent bright spot; a first extraction means that extracts a cell region from the morphological image or a fluorescent image among the fluorescent images; an in-focus plane specifying means that specifies a focal plane which is most in focus as an in-focus plane for each of the cell region extracted by the first extraction means; a coordinate specifying means that specifies a coordinate in the in-focus plane of the cell region for each of the cell region extracted by the first extraction means; a storage means that stores the in-focus plane and the coordinate for each of the cell region extracted by the first extraction means; a second extraction means that extracts a fluorescent bright spot region from a fluorescent image among the fluorescent images which is in a focal plane, among the focal planes, corresponding to the in-focus plane for each of the cell region extracted by the first extraction means; and a calculation means that calculates a luminance value or a number of the fluorescent nanoparticle in the fluorescent bright spot region.

### EFFECTS OF THE INVENTION

According to the present invention, it is possible to provide an image processing device, an in-focus position specifying method, and an in-focus position specifying program that specify the in-focus position from the fluorescent image and enable quantitative analysis of the expression amount of biological substance of the entire tissue sample.

### BRIEF DESCRIPTION OF DRAWINGS

FIG. 1 is a view showing a schematic configuration of an in-focus position specifying system according to the present invention.
FIG. 2 is a block diagram showing the functional configuration of an image processing device in FIG. 1.
FIG. 3 is a view showing an example of a bright field image.
FIG. 4 is a view showing an example of a fluorescent image.
FIG. 5 is a flowchart showing an image analysis process executed by the controller in FIG. 2.
FIG. 6 is a flowchart showing details of the process of step S2 in FIG. 5.
FIG. 7A is a view showing a bright field image.
FIG. 7B is a view showing an image in which a cell nucleus is extracted.
FIG. 8 is a flowchart showing details of the process of step S4 in FIG. 5.
FIG. 9 is a flowchart showing details of the process of step S5 in FIG. 5.
FIG. 10 is a flowchart showing details of the process of step S7 in FIG. 5.
FIG. 11A is a view showing partial images before image compositing.
FIG. 11B is a view showing a composited partial image.

### EMBODIMENTS FOR CARRYING OUT THE INVENTION

Hereinafter, embodiments for carrying out the present invention will now be described with reference to the drawings, which should not be construed to limit the present invention.

### <Configuration of In-Focus Position Specifying System 100>

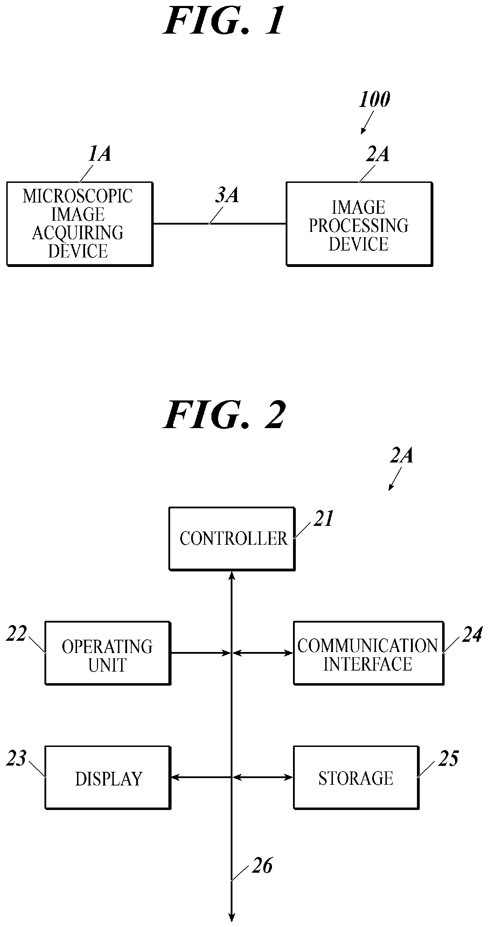

FIG. 1 illustrates an exemplary overall configuration of an in-focus position specifying system 100.

As illustrated in FIG. 1, in the in-focus position specifying system 100, a microscopic image acquiring device 1A and an image processing device 2A are connected via an interface such as a cable 3A, for transmission and reception of data.

The microscopic image acquiring device 1A may be connected to the image processing device 2A in any manner. For example, the microscopic image acquiring device 1A and the image processing device 2A may be connected through a local area network (LAN) or wireless communication.

The microscopic image acquiring device 1A is a known microscope provided with a camera, which acquires a microscopic image of a tissue section on a slide placed on a slide fixation stage, and transmits the acquired image to the image processing device 2A.

The microscopic image acquiring device 1A includes an irradiation means, an image forming means, an image capturing means, and a communication interface (I/F). The irradiation means includes a light source and a filter, and emits light toward the tissue section on the slide placed on the slide fixation stage. The image forming means includes an ocular and an objective lens, and generates an image with transmitted light, reflected light, or fluorescent light, which is emitted from the tissue section on the slide in response to the irradiated light. The image capturing means includes a charge coupled device (CCD) sensor or the like. The image capturing means is specifically a camera disposed in a microscope to capture an image formed on an image forming surface by the image forming means, and produce the digital image data of the microscopic image. The communication interface transmits the generated image data of the microscopic image to the image processing device 2A.

The microscopic image acquiring apparatus 1A includes a bright field unit combining the irradiation means and the image forming means suitable for bright field observation and a fluorescent unit combining the irradiation means and the image forming means suitable for fluorescence observation. The bright field/fluorescence observation can be switched by switching the units.

Any known microscope (for example, a phase contrast microscope, a differential interference microscope, an electron microscope, or the like) having a camera may be used as the microscopic image acquiring device 1A.

The microscopic image acquiring device 1A is not limited to the microscope having a camera. For example, a virtual microscope slide creating apparatus which scans a slide on a slide fixing stage of a microscope and obtains a microscopic image of the entire tissue section may be used (for example, see Japanese Patent Application Laid-Open Publication No. 2002-514319). According to the virtual microscope slide creating apparatus, there can be obtained image data with which the entire image of the tissue section on the slide can be viewed at once on a display.

The image processing device 2A analyzes the microscopic image transmitted from the microscopic image acquiring device 1A to specify the in-focus position for each cell in the tissue section of the observation target.

FIG. 2 shows a functional configuration example of the image processing device 2A.

As illustrated in FIG. 2, the image processing device 2A includes a controller 21, an operating unit 22, a display 23, a communication interface 24, and a storage 25, which are connected to each other through a bus 26.

The controller 21 includes a central processing unit (CPU), a random access memory (RAM), and the like. The controller 21 executes multiple processes in cooperation with a variety of programs stored in the storage 25 to control the overall operation of the image processing device 2A.

For example, the controller 21 executes an image analysis process in cooperation with an image processing program stored in the storage 25 and realizes the functions as a first extraction means, an in-focus plane specifying means, a coordinate specifying means, a second extraction means, a calculation means, and an image compositing means.

The operating unit 22 includes a keyboard including character input keys, numeral input keys and several functional keys, and a pointing device such as a mouse. The operating unit 22 outputs the pressing signal of the key which received the pressing operation with the keyboard and the operation signal by the mouse as input signals to the controller 21.

The display 23 includes a monitor, such as a cathode ray tube (CRT) display or a liquid crystal display (LCD). The display 23 displays a variety of windows in response to display signals input from the controller 21.

The communication interface 24 is an interface that allows data transmission and reception with external devices such as the microscopic image acquiring device 1A. The communication interface 24 realizes the function as an input means of the fluorescent image and the morphological image.

The storage 25 includes a hard disk drive (HDD) or a nonvolatile memory composed of a semiconductor, for example. The storage 25 stores a variety of programs and data as described above, the coordinates of cell in the cell region and the in-focus position for each cell to be descried later, and the like, and realizes the function as a storage means.

In addition, the image processing device 2A may include a LAN adaptor and a router to be connected to external devices through a communication network such as a LAN.

### <Images>

In the embodiment, for example, the image processing device 2A preferably performs analysis by using fluorescent images representing, with fluorescent bright spots, expression of a specific biological substance in cells, and morphological images (for example, bright field images) representing morphology of the entire cell and morphology of predetermined structure in cells such as cell nucleus and cell membrane, which were transmitted from the microscopic image acquiring device 1A.

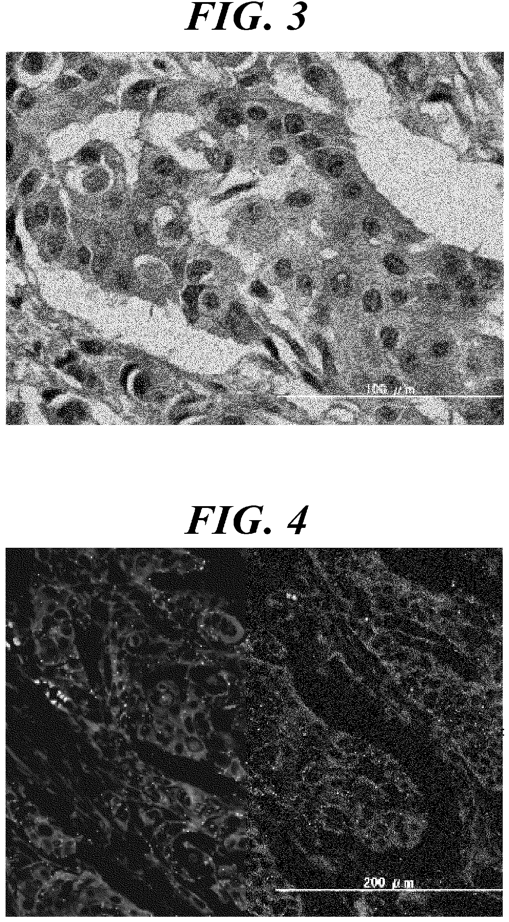

The "bright field image" is, for example, a microscopic image acquired by, in the microscopic image acquiring device 1A, forming and capturing an enlarged image of a tissue section stained with a reagent for hematoxylin staining (H-staining reagent) or a reagent for hematoxylin-eosin staining (HE-staining reagent) in a bright field, and a cell morphological image representing the morphology of cell in the tissue section. FIG. 3 shows an example of the bright field image. Hematoxylin (H) is a bluish violet dye and stains cell nuclei, bony tissue, a portion of cartilaginous tissue, serous components, and the like (basophilic tissue and the like). Eosin (E) is a red to pink dye and stains cytoplasm, connective tissue of soft tissue, red blood cells, fibrin, endocrine granules and the like (acidophilic tissue and the like).

Examples of the morphological image of cell(s) may include, in addition to the bright field image, a fluorescent image obtained by capturing fluorescence emitted from a fluorescent staining reagent which is used for staining a tissue section and which can specifically stain a cell structure to be diagnosed. Examples of the fluorescent staining reagent used for obtaining the morphological image include DAPI staining reagent for staining cell nuclei, Papanicolaou staining reagent for staining cytoplasm, and the like. Examples of the morphological image also include a phase difference image, a differential interference image, an electron microscope image, and the like.

A "fluorescent image" representing expression of a specific biological substance in a cell with a fluorescent bright spot is a microscopic image obtained by forming and capturing an enlarged image of the fluorescence emitted by the fluorescent substance by irradiation of the tissue section stained with a fluorescent staining reagent with excitation light having a predetermined wavelength in the microscopic image acquiring device 1A. FIG. 4 shows an example of the fluorescent image.

The fluorescent staining reagent indicates fluorescent nanoparticles which specifically bond and/or react with the specific biological substance in the present embodiment. As will be described in detail later, "fluorescent nanoparticles" are nano-sized particles which emit fluorescence in response to the irradiation with excitation light. The particles can emit fluorescence having a sufficient intensity for representing each molecule of the specific biological substance as a bright spot.

Preferably used fluorescent nanoparticles include quantum dots (semiconductor nanoparticles) or fluorescent substance-containing nanoparticles. Preferably used fluorescent nanoparticles have an emission wavelength within the sensitivity range of the image capturing element of the microscopic image acquiring device 1A, specifically, an emission wavelength of 400 to 700 nm.

### <Fluorescent Staining Reagent and Staining Method>

Hereinafter, a fluorescent staining reagent and a staining method of a tissue section using the fluorescent staining reagent are described. The fluorescent staining reagent is used for obtaining the fluorescent image representing the expression of the specific biological substance expressed specifically with respect to a cell with the fluorescent bright spot.

### (1) Fluorescent Substance

Examples of the fluorescent substance used in the fluorescent staining reagent include a fluorescent organic dye and a quantum dot (semiconductor particles). Preferably, the substance exhibits emission of visible to near infrared rays having a wavelength within the range from 400 to 1100 nm when excited by ultraviolet to near infrared rays having a wavelength within the range from 200 to 700 nm.

Examples of the fluorescent organic dye include fluorescein dye molecules, rhodamine dye molecules, Alexa Fluor (manufactured by Invitrogen Corporation) dye molecules, BODIPY (manufactured by Invitrogen Corporation) dye molecules, cascade dye molecules, coumarin dye molecules, eosin dye molecules, NBD dye molecules, pyrene dye molecules, Texas Red dye molecules and cyanine dye molecules.

Specific examples thereof include 5-carboxy-fluorescein, 6-carboxy-fluorescein, 5,6-dicarboxy-fluorescein, 6-carboxy-2',4,4',5',7,7'-hexachlorofluorescein, 6-carboxy-2',4,7,7'-tetrachlorofluorescein, 6-carboxy-4',5'-dichloro-2',7'-dimethoxyfluorescein, naphthofluorescein, 5-carboxy-rhodamine, 6-carboxy-rhodamine, 5,6-dicarboxy-rhodamine, rhodamine 6G, tetramethylrhodamine, X-rhodamine, and Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Al'xa Fluor 568, Alexa Fluor 594, Alexa Fluor 610, Alexa Fluor 633, Alexa Fluor 635, Alexa Fluor 647, Alexa Fluor 660, Alexa Fluor 680, Alexa Fluor 700, Alexa Fluor 750, BODIPY FL, BODIPY TMR, BODIPY 493/503, BODIPY 530/550, BODIPY 558/568, BODIPY 564/570, BODIPY 576/589, BODIPY 581/591, BODIPY 630/650, BODIPY 650/665 (the above are manufactured by Invitrogen Corporation), methoxycoumalin, eosin, NBD, pyrene, Cy5, Cy5.5 and Cy7. These can be used individually, or used by mixing a plurality of kinds thereof.

Usable examples of the quantum dot include quantum dots respectively containing, as a component, II-VI compounds, III-V compounds, and IV elements (called "II-VI quantum dot", "III-V quantum dot" and "IV quantum dot", respectively). These can be used individually, or used by mixing a plurality of kinds thereof.

Specific examples thereof include but are not limited to CdSe, CdS, CdTe, ZnSe, ZnS, ZnTe, InP, InN, InAs, InGaP, GaP, GaAs, Si and Ge.

A quantum dot having a core of any of the above quantum dots and a shell provided thereon can also be used. Hereinafter, as a notation for the quantum dot having a shell, when the core is CdSe and the shell is ZnS, the quantum dot is noted as CdSe/ZnS.

Usable examples of the quantum dot include but are not limited to CdSe/ZnS, CdS/ZnS, InP/ZnS, InGaP/ZnS, Si/SiO₂, Si/ZnS, Ge/GeO₂, and Ge/ZnS.

A quantum dot surface-treated with an organic polymer or the like may be used as needed. Examples thereof include CdSe/ZnS having a surface carboxy group (manufactured by Invitrogen Corporation) and CdSe/ZnS having a surface amino group (manufactured by Invitrogen Corporation).

### (2) Fluorescent Substance-containing Nanoparticle

The "fluorescent substance-containing nanoparticles" is nanoparticles containing therein the fluorescent substance as described above, and particularly, indicates nanoparticles in which the fluorescent substance is dispersed. The fluorescent substance and the nanoparticles may or may not be chemically bonded with each other.

The material composing the nanoparticles is not particularly limited, and examples thereof include silica, polystyrene, polyactate acid, melamine, and the like.

The fluorescent substance-containing nanoparticles can be produced by a publically-known method.

For example, fluorescent organic dye-containing silica nanoparticles can be synthesized by referring to the synthesis of FITC-containing silica nanoparticles described in Langmuir, vol. 8, page 2921 (1992). A variety of fluorescent organic dye-containing silica nanoparticles can be synthesized by using any desired fluorescent organic dye instead of FITC.

Quantum dot-containing silica nanoparticles can be synthesized by referring to the synthesis of CdTe-containing silica nanoparticles described in New Journal of Chemistry, vol. 33, page 561 (2009).

Fluorescent organic dye-containing polystyrene nanoparticles can be produced by using a copolymerization method using an organic dye having a polymerizable functional group described in U.S. Patent No. 4,326,008 (1982) or a method of impregnating a fluorescent organic dye into polystyrene nanoparticles described in U.S. Patent No. 5,326,692 (1992).

Quantum dot-containing polymer nanoparticles can be produced by using the method of impregnating a quantum dot into polystyrene nanoparticles described in Nature Biotechnology, vol. 19, page 631 (2001).

The average particle diameter of the fluorescent substance-containing nanoparticles is not particularly limited, and preferably, from about 30 to 800 nm. A coefficient of variation (= (standard deviation / average value) × 100%) indicating variation of the particle diameter is not particularly limited, but preferably 20% or less.

The average particle diameter is obtained as follows: capturing the electronic microscope picture using the scanning electron microscope (SEM), measuring the cross sectional area of a sufficient number of particles, and obtaining the diameter of a circle having the area of each measured value as the particle diameter. In the present embodiment, the average particle diameter is to be a calculated average of particle diameters from 1000 particles. The coefficient of variation is also to be a value calculated from particle diameter distribution of 1000 particles.

### (3) Bonding of Biological Substance-Recognizing Portion and Fluorescent Nanoparticles

In the explanation of the embodiment, fluorescent nanoparticles and the biological substance-recognizing portion are directly bonded with each other in advance and used as the fluorescent staining reagent which specifically bonds and/or reacts with a specific biological substance. A "biological substance-recognizing portion" is a portion which specifically bonds and/or reacts with a specific biological substance.

The specific biological substance is not particularly limited as long as there exists a substance specifically bonding with the specific biological substance. Representative examples of the substance include protein (peptide), nucleic acid (oligonucleotide, polynucleotide), and the like.

Therefore, examples of the biological substance-recognizing portion include an antibody which recognizes the protein as an antigen, another protein which specifically bonds with the protein, nucleic acid including a base sequence which hybridizes with the nucleic acid, and the like.

Specific examples of the biological substance-recognizing portion include anti-HER2 antibody which specifically bonds with the HER2 which is a protein on the surface of the cell, anti-ER antibody which specifically bonds with the estrogen receptor (ER) in the cell nucleus, anti-actin antibody which specifically bonds with the actin forming the cytoskeleton, and the like.

Among the above, anti-HER2 antibody and anti-ER antibody bonded to the fluorescent nanoparticles (fluorescent staining reagent) are preferable because they can be used for selecting drug administration to treat breast cancer.

The bonding form between the biological substance-recognizing portion and the fluorescent nanoparticles is not particularly limited, and examples include, covalent bond, ionic bond, hydrogen bond, coordinate bond, physical adsorption, chemical adsorption, and the like. Bonding with a strong bonding force such as covalent bond is preferable due to the stability of bonding.

There can be an organic molecule connecting the biological substance-recognizing portion and the fluorescent nanoparticles. For example, in order to suppress non-specific absorption with the biological substance, a polyethyleneglycol chain, such as SM (PEG) 12 manufactured by Thermo Scientific, can be used.

When the biological substance-recognizing portion is bonded to the fluorescent substance-containing silica nanoparticles, the same process can be applied for either case where the fluorescent substance is the fluorescent organic dye or the quantum dot.

For example, a silane coupling agent which is a compound widely used for bonding inorganic material and organic material can be used. The silane coupling agent is a compound including an alkoxysilyl group providing a silanol group with hydrolysis in one end of the molecule and a functional group such as carboxy group, amino group, epoxy group, aldehyde group, and the like in the other end, and bonds with the inorganic material through an oxygen atom of the silanol group.

Specific examples include mercaptopropyl triethoxysilane, glycidoxypropyl triethoxysilane, aminopropyl triethoxysilane, silane coupling agent including polyethylene glycol chain (for example, PEG-silane no. SIM6492.7 manufactured by Gelest Inc.), and the like.

When the silane coupling agent can be used, two or more kinds can be used together.

Well-known methods can be used as the reaction method between the fluorescent organic dye-containing silica nanoparticles and the silane coupling agent.

For example, the obtained fluorescent organic dye-containing silica nanoparticles can be dispersed in pure water, the aminopropyl triethoxysilane can be added, and the above reaction can be performed at room temperature for 12 hours. After the reaction ends, by centrifugal separation or filtration, it is possible to obtain fluorescent organic dye-containing silica nanoparticles having a surface modified with the aminopropyl group. Next, the amino group is reacted with the carboxy group in the antibody so that the antibody can bond with the fluorescent organic dye-containing silica nanoparticles through amide bond. If necessary, condensing agent such as EDC (1-Ethyl-3-[3-Dimethylaminopropyl] carbodiimide Hydrochloride: manufactured by Pierce (Registered Trademark)) can also be used.

If necessary, a linker compound including a portion which can directly bond with the fluorescent organic dye-containing silica nanoparticles modified with the organic molecule and a portion which can bond with the molecular target substance can be used. For example, when sulfo-SMCC (Sulfosuccinimidyl 4[N-maleimidomethyl]-cyclohexane-1-carboxylate: manufactured by Pierce) which has a portion which selectively reacts with the amino group and a portion which selectively reacts with the mercapto group is used, the amino group of the fluorescent organic dye-containing silica nanoparticles modified with aminopropyl triethoxysilane and the mercapto group in the antibody are bonded, and with this, the fluorescent organic dye-containing silica nanoparticles bonded with the antibody is made.

When the biological substance-recognizing portion is bonded to the fluorescent substance-containing polystyrene nanoparticles, the same process can be applied either the fluorescent substance is the fluorescent organic dye or the quantum dot. In other words, by impregnating the fluorescent organic dye and the quantum dot in the polystyrene nanoparticles with the functional group such as the amino group, it is possible to obtain the fluorescent substance-containing polystyrene nanoparticles with the functional group, and then by using the EDC or the sulfo-SMCC, the fluorescent substance-containing polystyrene nanoparticles bonded with the antibody is made.

Examples of biological substance-recognizing portion include the antibody which recognizes the following specific antigen, such as M. actin, M.S. actin, S.M. actin, ACTH, Alk-1, α1-antichymotrypsin, α1-antitrypsin, AFP, bcl-2, bcl-6, β-catenin, BCA 225, CA19-9, CA125, calcitonin, calretinin, CD1a, CD3, CD4, CD5, CD8, CD10, CD15, CD20, CD21, CD23, CD30, CD31, CD34, CD43, CD45, CD45R, CD56, CD57, CD61, CD68, CD79a, "CD99, MIC2", CD138, chromogranin, c-KIT, C-MET, collagen type IV, Cox-2, cyclin D1, keratin, cytokeratin (high molecular mass), pankeratin, pankeratin, cytokeratin 5/6, cytokeratin 7, cytokeratin 8, cytokeratin 8/18, cytokeratin 14, cytokeratin 19, cytokeratin 20, CMV, E-cadherin, EGFR, ER, EMA, EBV, VIII factor related antigen, fassin, FSH, galectin-3, gastrin, GFAP, glucagon, glycophorin A, granzyme B, hCG, hGH, helicobacter pyroli, HBc antigen, HBs antigen, hepatocyte specific antigen, HER2, HSV-I, HSV-II, HHV-8, IgA, IgG, IgM, IGF-1R, inhibin, insulin, kappa L chain, Ki67, lambda L chain, LH, lysozyme, macrophage, melan A, MLH-1, MSH-2, myeloperoxidase, myogenin, myoglobin, myosin, neurofilament, NSE, p27 (Kip1), p53, p53, p63, PAX 5, PLAP, pneumocystis calini, podoplanin (D2-40), PGR, prolactin, PSA, prostatic acid phosphatase, Renal Cell Carcinoma, S100, somatostatin, spectrin, synaptophysin, TAG-72, TdT, thyroglobulin, TSH, TTF-1, TRAcP, tryptase, villin, vimentin, WT1, Zap-70, and the like.

The fluorescent nanoparticles may be directly connected to the biological substance-recognizing portion as described above. Otherwise, as in the indirect method in publically-known immunological staining, the fluorescent nanoparticles may be bonded to the biological substance-recognizing portion indirectly in the staining step. Specifically, for example, the tissue sample is reacted with a biotinylated primary antibody with the specific biological substance as antigen, further reacted with a staining reagent including the fluorescent nanoparticles modified by streptavidin, so that the staining is performed by the specific bonding of streptavidin and biotin to form a complex. Furthermore, the tissue sample may be reacted with a primary antibody with the specific protein as an antigen, further reacted with a secondary biotinylated antibody with the primary antibody as an antigen, reacted with the fluorescent nanoparticles modified by streptavidin for staining.

### (4) Staining Method

The method of preparing the tissue section is not particularly limited. A tissue section made by publically-known methods can be used. The staining method described below is not limited to a pathological tissue section, and can be applied to cultured cells.

### (4.1) Paraffin Removing Step

The tissue section is immersed in a container with xylene, and paraffin is removed. The temperature is not particularly limited, and the processing can be performed at room temperature. Preferably, the immersing time is 3 minutes or more and 30 minutes or less. The xylene can be changed during the immersion as necessary.

Next, the tissue section is immersed in a container with ethanol, and the xylene is removed. The temperature is not particularly limited, and the processing can be performed at room temperature. Preferably, the immersing time is 3 minutes or more to 30 minutes or less. The ethanol can be changed during the immersion as necessary.

Next, the tissue section is immersed in a container with water to remove the ethanol. The temperature is not particularly limited, and the processing can be performed at room temperature. Preferably, the immersing time is 3 minutes or more and 30 minutes or less. The water can be changed during the immersion as necessary.

### (4.2) Activating Processing

Activating processing of the biological substance in the tissue section is performed according to publically-known methods.

The activating conditions are not specifically set, and examples of liquid for activation that can be used include, 0.01 M citric acid buffered solution (pH 6.0), 1 mM EDTA solution (pH 8.0), 5% urea, 0.1 M tris-hydrochloric acid buffered solution. Examples of the heating device that can be used include autoclave, microwave, pressure pan, water bath, and the like. The temperature is not particularly limited, and the processing can be performed at room temperature. The processing can be performed at a temperature of 50 to 130 °C and the amount of time that the processing is performed can be 5 to 30 minutes.

Next, the tissue section after the activating processing is immersed in the container with PBS (Phosphate Buffered Saline), and cleaning is performed. The temperature is not limited, and the processing can be performed at room temperature. Preferably, the immersing time is 3 minutes or more to 30 minutes or less. The PBS can be changed during the immersion as necessary.

### (4.3) Staining Using Fluorescent Staining Reagent

The PBS dispersion liquid of the fluorescent staining reagent is placed on the tissue section and reacted with the biological substance in the tissue section.

By changing the biological substance-recognizing portion in the fluorescent staining reagent, staining can be applied to various biological substances. When the fluorescent nanoparticles bonded with a plurality of kinds of biological substance-recognizing portion are used as the fluorescent staining reagent, the fluorescent nanoparticles PBS dispersion liquid of each of the above can be mixed in advance, or the liquid can be sequentially placed on the tissue section separately. The temperature is not particularly limited, and the processing can be performed at room temperature. Preferably, the reacting time is 30 minutes or more to 24 hours or less.

Preferably, a publically-known blocking agent such as BSA included in PBS is dropped before staining with the fluorescent staining reagent.

Next, the tissue section after the staining is immersed in the container with PBS, and the unreacted fluorescent nanoparticles are removed. The temperature is not particularly limited, and the processing can be performed at room temperature. Preferably, the immersing time is 3 minutes or more to 30 minutes or less. The PBS can be changed during the immersion as necessary. A cover glass is placed on the tissue section to be sealed. A commercially available sealing agent can be used as necessary.

The HE staining or the like to obtain the morphological image is performed in any step before sealing with the cover glass.

### (5) Obtaining Fluorescent Image

The microscopic image acquiring device 1A is used to obtain the microscopic image (fluorescent image) of the stained tissue section. The excitation light source and the optical filter for fluorescence detection are suitably selected according to the absorption maximum wavelength and the fluorescent wavelength of the fluorescent substance used in the fluorescent staining reagent.

### <Operation of In-Focus Position Specifying System 100>

Hereinafter, the operation of obtaining the above-described fluorescent image and bright field image and performing analysis in the in-focus position specifying system 100 will be described.

The present embodiment will be described by taking, as an example, a case of observing the tissue sample stained by using the staining reagent including the fluorescent substance-containing nanoparticles bonded to the biological substance-recognizing portion recognizing a specific protein (for example, Ki67 protein or the like in the breast cancer tissue, hereinafter referred to as a specific protein). However, the present invention is not limited to this. In the present invention, a plurality of types of biological substances can be stained by using fluorescent nanoparticles having different light emission properties, and the observation thereof can be performed on a same window.

The operator first stains the tissue sample by using two types of staining reagents that are the HE staining reagents and the staining reagent having, as the fluorescent labeling material, the fluorescent substance-containing nanoparticles bonded to the biological substance-recognizing portion recognizing the specific protein.

Thereafter, bright field images and fluorescent images are obtained with the microscopic image acquiring device 1A by the following steps (a1) to (a5).
(a1) The operator mounts the tissue sample stained with the hematoxylin staining reagent and the staining reagent including fluorescent substance-containing nanoparticles on a slide, and places the slide on a slide fixing stage of the microscopic image acquiring device 1A.
(a2) The bright field unit is set, the capturing magnification and focus are adjusted, and the observation target region in the tissue is positioned in the visual field.
(a3) Capturing is performed with the image capturing means while shifting the focal plane at predetermined intervals (for example, 0.5um) in the height direction (Z direction) of the tissue sample from the upper surface to the lower surface of the tissue sample, to generate image data of a plurality of bright field images, and the image data is transmitted to the image processing device 2A.
(a4) The unit is changed to the fluorescent unit.
(a5) Capturing of the fluorescent images of focal planes corresponding to the focal planes at the time of capturing of the bright field images is performed while shifting the focal plane in the height direction of the tissue sample with the image capturing means, without changing the visual field and the capturing magnification, to generate image data of a plurality of fluorescent images. The image data is transmitted to the image processing device 2A.

When a plurality of types of biological substances are stained, the step of the above (a5) is repeated. As for the excitation light and filter which are used for obtaining each fluorescent image, the combination suitable for the light emission property is selected as needed.

The image analysis process based on the bright field images and the fluorescent images is executed in the image processing device 2A.

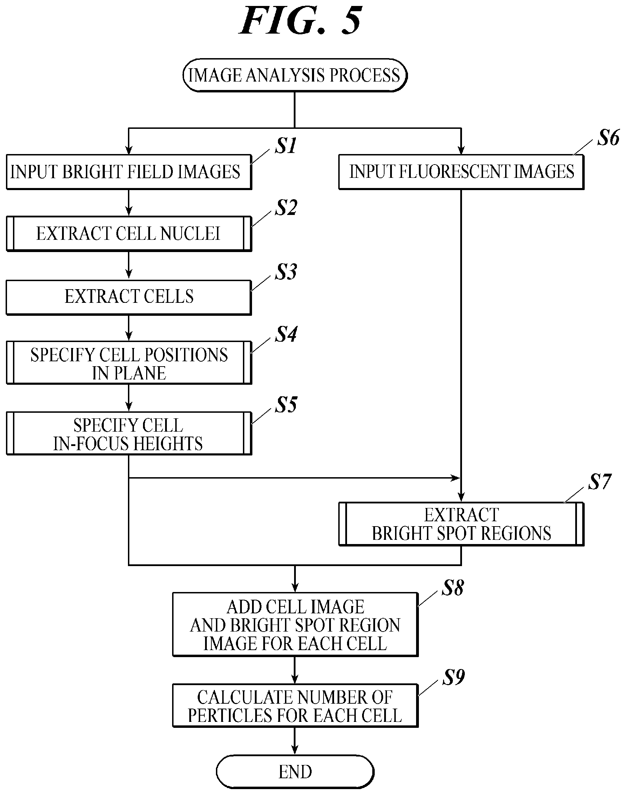

FIG. 5 shows a flowchart of the image analysis process in the image processing device 2A. The image analysis process shown in FIG. 5 is executed by cooperation between the controller 21 and the program stored in the storage 25.

First, when all of the plurality of bright field images captured at predetermine intervals in the Z direction are input from the microscopic image acquiring device 1A by the communication interface 24 (step S1), extraction of cell nucleus region from each of the bright field images is performed (step S2).

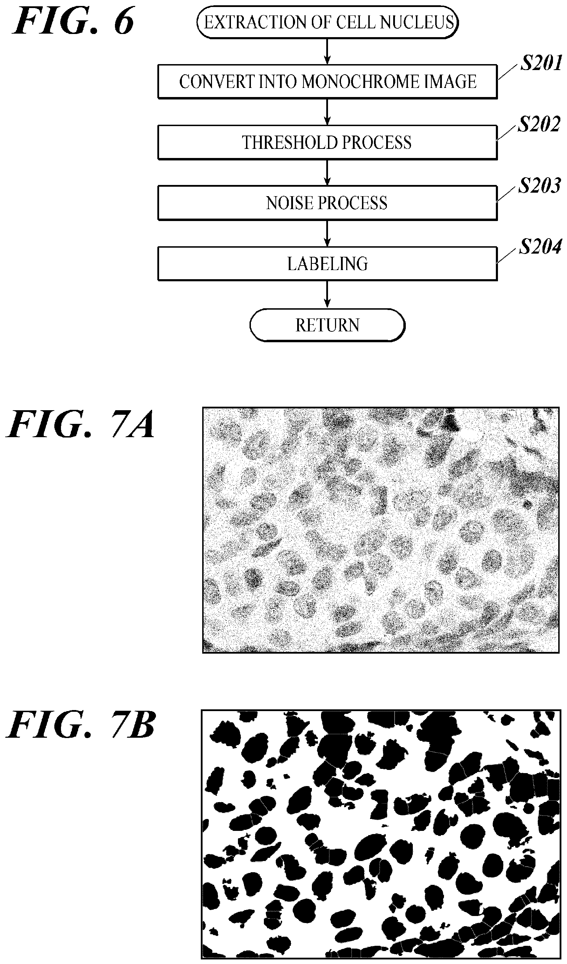

FIG. 6 shows the detailed flow of the process in step S2. The process of step S2 is executed by cooperation between the controller 21 and the program stored in the storage 25.

In step S2, conversion to the monochrome image is performed for each of the bright field images (step S201). FIG. 7A shows an example of the bright field image.

Next, a threshold process is performed by using a threshold which is determined in advance to the monochrome image, and the value of each pixel is binarized (step S202).

A noise process is then performed (step S203). The noise process can be performed particularly by performing a closing process to the binary image. The closing process is a process of performing an expansion process and then performing a shrinking process the same number of times. The expansion process is a process of replacing a pixel of interest with a white pixel when there is one or more white pixel(s) in the range of n x n pixels (n is an integer greater than or equal to 2) with respect to the pixel of interest. The shrinking process is a process of replacing a pixel of interest with a black pixel when there is one or more black pixel(s) in the range of n x n pixels with respect to the pixel of interest. By the closing process, it is possible to remove small regions of noise and the like. FIG. 7B shows an example of the image after the noise process. As shown in FIG. 7B, an image having cell nuclei extracted (cell nucleus image) is generated after the noise process.

Next, the labeling process is performed to the image after the noise process, and a label is provided to each extracted cell nucleus (step S204). The labeling process is a process of identifying an object in the image by providing a same label (number) to connected pixels. By the labeling process, it is possible to provide a label identifying each cell nucleus from the image after the noise process. The process of step S2 is executed for all of the plurality of bright field images which were input.

In step S3, extraction of cell regions from each of the bright field images is performed. The extraction of cell regions is performed by recognizing each cell nucleus and cutting out the cell membrane as a border. The value of color, size, roundness or the like of the cell nucleus is used for recognizing the cell nucleus, for example. The process of step S3 is executed for all of the plurality of bright field images which were input.

In step S4, specification of cell positions in the plane is performed for each cell.

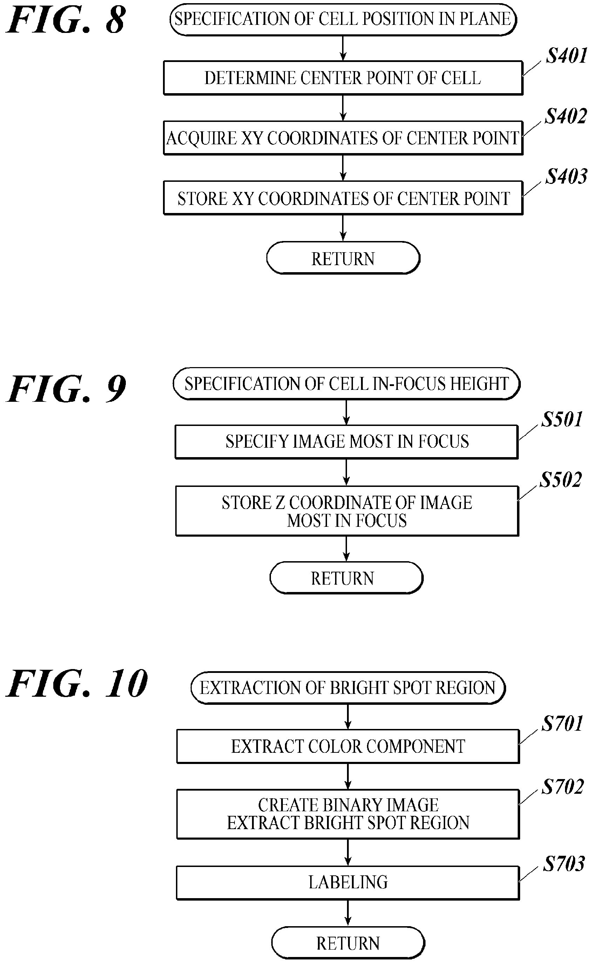

FIG. 8 shows the detailed flow of the process in step S4. The process of step S4 is executed by cooperation between the controller 21 and the program stored in the storage 25.

First, a center point is determined for each individual cell region extracted in step S3 (step S401). The center point of the cell region is, for example, a point which is a center of a rectangle surrounding the cell in a manner of contacting the outer edge of the cell.

Next, the coordinates on the image plane (hereinafter, described as XY coordinates) of the center point determined in step S401 is obtained (step S402), and the XY coordinates are stored in the storage 25 (step S403). The process of step S4 does not need to be executed for all of the plurality of bright field images which were input, as long as the process of step S4 is executed for the bright field image for which the center point and its XY coordinates can be specified for each cell.

Since the position in the plane of each cell is stored by the process of step S4, at the time of specifying the in-focus height for each cell to be described later, it is possible to continue the analysis without losing the sight of the cells even when the images of focal planes having different heights in the Z direction are treated.

In step S5, specification of in-focus position is performed for each cell.

FIG. 9 shows the detailed flow of the process in step S5. The process of step S5 is executed by cooperation between the controller 21 and the program stored in the storage 25.

For each of the cell regions extracted in step S3, the image which is most in focus is specified from among the plurality of bright field images for each cell region (step S501). For example, the size of cell nucleus (diameter, area, circumferential length, or the like) or the contrast can be used to specify the image in which the cell is most in focus. At this time, since the XY coordinates of each cell are stored in the storage 25 in step S4, it is possible to execute the process without losing the sight of the cell even when the shape or the size of cell changes between the images having different heights in the Z direction.

The coordinate in the Z direction (hereinafter, described as Z coordinate) of the image which is most in focus is then stored in the storage 25 (step S502).

When the fluorescent images from the microscopic image acquiring device 1A are input by the communication interface 24 (step S6), the bright spot regions are extracted from the fluorescent images (step S7). The process of step S7 is executed for the image in which each individual cell is most in focus, which was specified in step S5.

FIG. 10 shows the detailed flow of the process in step S7. The process of step S7 is executed by cooperation between the controller 21 and the program stored in the storage 25.

In step S7, the color component corresponding to the wavelength of fluorescent bright spot is extracted from the fluorescent image (step S701). In step S701, for example, when the light emission wavelength of fluorescent particle is 550nm, only the fluorescent bright spot having the wavelength component is extracted as the image.

The threshold process is performed to the extracted image to generate a binary image, and the bright spot region having the fluorescent bright spot at the center is extracted (step S702).

Any noise removal process of removing cell autofluorescence, other unnecessary signal components or the like may be performed before the threshold process, and low-pass filters such as a Gaussian filter and high-pass filters such as a quadratic differential are preferably used.

The labeling process is then performed to the bright spot region to provide a label to each of the extracted bright spot regions (step S703).

After the processes of steps S5 and S7 are finished, returning to the process of FIG. 5, an addition process of the cell image and the bright spot region image is performed for each cell (step S8). In the addition process in step S8, the fluorescent image for which the bright spot region was extracted in step S7 and the bright field image having the same Z coordinate as the Z coordinate of the fluorescent image are added to each other. That is, by the process of step S8, there is obtained a second fluorescent image in which the bright field image being most in focus for the individual cell and the fluorescent image displaying the bright spots of fluorescent nanoparticles bonded to the surface of the cell are superposed on each other.

Next, the number of fluorescent nanoparticles for each cell is calculated by using the second fluorescent image composited in step S8 (step S9). The number of fluorescent nanoparticles for each cell can be calculated by, for example, counting the "bright spots existing inside the cell membrane". Alternatively, a certain region outside the cell membrane may be included by providing a reference such as "bright spots existing inside ten pixels outside the cell membrane", for example. The image analysis system ends by the above process.

As described above, the in-focus position specifying system 100 according to the present embodiment includes: a microscopic image acquiring device 1A that captures morphological images and fluorescent images while changing the focal plane in the height direction of the tissue sample; and an image processing device 2A that performs image processing to the captured morphological images and the fluorescent images. The image processing device includes a communication interface, a controller 21, and a storage 25. The communication interface is as an input means to input a plurality of bright field images and a plurality of fluorescent images. The controller 21 functions as a first extraction means that extracts cell regions from the bright field image, an in-focus plane specifying means that specifies the focal plane which is most in focus for each cell region, a coordinate specifying means that specifies the coordinates in the focal plane of the cell region for each cell region, a second extraction means that extracts the fluorescent bright spot region from the fluorescent image in the focal plane which is most in focus for each cell region, and a calculation means that calculates the luminance value or the number of fluorescent dye accumulated particles in the fluorescent bright spot region. The storage 25 is as a storage means that stores the focal plane and the coordinates. Accordingly, by the in-focus position specifying system 100 according to the present invention, it is possible to specify the in-focus position for each cell region. That is, since in-focus positions for all the cells in the tissue sample can be specified, it is possible to accurately perform quantitative analysis of the biological substance expression amount in the entire tissue sample.

The in-focus position specifying system 100 according to the present invention recognizes a cell nucleus from the bright field image, and extracts the cell region by using a value of color, size, roundness, or the like of the cell nucleus. Since the cell nucleus can be easily recognized compared to the other regions in the cell, the cell nucleus is suitable for extraction of cell.

The in-focus position specifying system 100 according to the present invention calculates the number of fluorescent nanoparticles for each extracted cell region. Accordingly, by adding up the value of each cell region, it is possible to calculate the number of fluorescent nanoparticles in the entire tissue sample.

By the in-focus position specifying system 100 according to the present invention, a partial image cutting out a cell region from the bright field image which is most in focus is created for each cell, fluorescence compositing is performed to the partial images of all the cell regions, and thereby a single fluorescent image can be reconstituted.

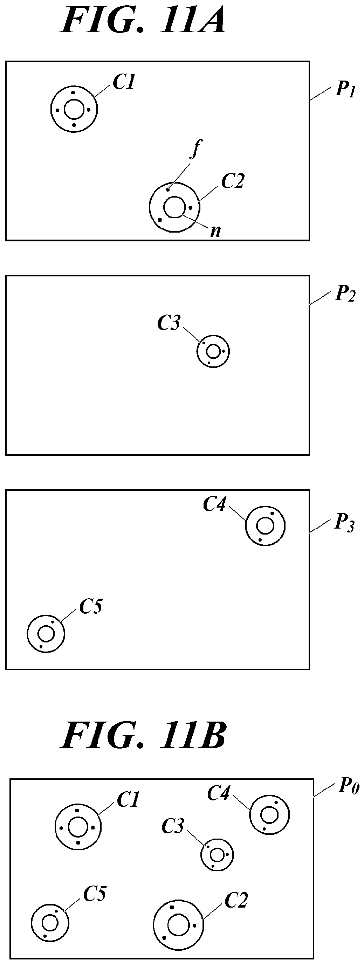

To be specific, in step S8 of FIG. 5, an image of adding the cell image and the bright spot region image is obtained for each cell. For example, in FIG. 11A, the cells C1 and C2 are in focus in the bright field image P1, the cell C3 is in focus in the bright field image P2, and the cells C4 and C5 are in focus in the bright field image P3 (in the drawing, n indicates the cell nucleus, and f indicates the fluorescent bright spot). The cell regions are extracted from these bright field images, and the bright spot region images are added to create partial images. Then, as shown in FIG. 11B, the partial images for respective cells are reconstituted on a same plane on the basis of the XY coordinates by the controller 21 as an image compositing means. Thus, it is possible to obtain a single fluorescent image P0 in which all the cell regions are in focus, and the fluorescent bright spots are drawn in the respective cell regions.

Alternatively, immunostaining is performed to the tissue section with the fluorescent nanoparticles, the cell nucleus is stained with the fluorescent dye such as DAPI, and a plurality of fluorescent images are captured by changing the focal plane as mentioned above. The cell regions are extracted from the captured fluorescent images, and the Z coordinate which is most in focus is derived for each cell region, and thereby the same effect can be obtained. In this case, the fluorescent image also achieves the function as the morphological image.

Thus, various types of analysis such as quantification of the objective protein can be executed on the basis of a single image, which is efficient.

### [Other Embodiments]

Though the specific description has been made based on the embodiment according to the present invention as described above, the above embodiment is a preferred example of the present invention, and the present invention is not limited to this.

In the above embodiment, as the quantitative analysis of biological substance, the number of fluorescent nanoparticles is calculated in step S9 of FIG. 5. However, the quantitative analysis is not limited to this, and the expression amount of biological substance can be quantified by calculating the luminance value in the fluorescent bright spot region.

In the above embodiment, the extraction of cell is executed by recognizing the cell nucleus. However, the extraction of cell is not limited to this, and can be executed by recognizing other organs such as the cell membrane, for example.

In the above embodiment, the bright field images are a plurality of images having different focal planes in the height direction of the tissue sample. However, the bright field image does not need to be a plurality of images, but may be a single image as long as the cell in the field can be extracted.

In the above embodiment, when the shift of in-focus position between the bright field image and the fluorescent image is known in advance, the number of fluorescent nanoparticles or the luminance value may be calculated by extracting the bright spot from the fluorescent image at the height considering the offset value to the in-focus position of the bright field image.

In the above embodiment, the description has been made for a case of quantifying the expression amount of a single type of biological substance as an example of the specific protein. However, the specific protein is not limited to this, and a plurality of types of biological substances can be quantified by using fluorescent nanoparticles having different light emission properties. For example, in the breast cancer tissue, classification of subtype of the breast cancer can be performed by analyzing the expression of hormone receptor (estrogen receptor (ER) and progesterone receptor (PgR)), HER2 and Ki67.

In the above embodiment, the shape of cell is used as the cell feature amount. However, the cell feature amount is not limited to this, and the shape of cell nucleus may be extracted as the cell feature amount. Thus, by detecting the atypism such as hypertrophy of cell nucleus in the cancer cell, for example, it is possible to perform classification into positive cell or negative cell.

In the above description, examples of using HDD, a semiconductor nonvolatile memory or the like as a computer readable medium for the program according to the present invention have been disclosed, but the medium is not limited to these examples. For other computer readable media, a portable recording medium such as CD-ROM can be applied. Moreover, as a medium that provides data of the program according to the present invention via a communication line, a carrier wave may be applied.

Besides, a detailed configuration and a detailed operation of each device constituting the in-focus position specifying system 100 can also be appropriately modified within a range that does not depart from the scope of the present invention.

### INDUSTRIAL APPLICABILITY

The present invention is applicable to an image processing device, an in-focus position specifying method, and an in-focus position specifying program.

### EXPLANATION OF REFERENCE NUMERALS

1A microscopic image acquiring device
2A image processing device
3A cable
21 controller (input means, first extraction means, in-focus plane specifying means, coordinate specifying means, second extraction means, calculation means, and image compositing means)
22 operating unit
23 display
24 communication interface (input means)
25 storage (storage means)
26 bus
100 in-focus position specifying system

## Claims

1. An image processing device comprising:
an input means to input a morphological image and a plurality of fluorescent images, the morphological image representing a morphology of a cell in a tissue sample in which a biological substance is stained with a fluorescent nanoparticle, the biological substance being a single type of biological substance or a plurality of types of biological substances, and the plurality of fluorescent images having focal planes which are different at a predetermined interval in a height direction of the tissue sample in a same range as a range of the morphological image and representing expression of the biological substance in the tissue sample with a fluorescent bright spot;
a first extraction means that extracts a cell region from the morphological image or a fluorescent image among the fluorescent images;
an in-focus plane specifying means that specifies a focal plane which is most in focus as an in-focus plane for each of the cell region extracted by the first extraction means;
a coordinate specifying means that specifies a coordinate in the in-focus plane of the cell region for each of the cell region extracted by the first extraction means;
a storage means that stores the in-focus plane and the coordinate for each of the cell region extracted by the first extraction means;
a second extraction means that extracts a fluorescent bright spot region from a fluorescent image among the fluorescent images which is in a focal plane, among the focal planes, corresponding to the in-focus plane for each of the cell region extracted by the first extraction means; and
a calculation means that calculates a luminance value or a number of the fluorescent nanoparticle in the fluorescent bright spot region.

2. The image processing device according to claim 1, wherein
the morphological image is a plurality of morphological images having focal planes which are different at a predetermined interval in the height direction of the tissue sample and representing the morphology of the cell, and
the fluorescent images are a plurality of fluorescent images representing, with the fluorescent bright spot, the expression of the biological substance in focal planes corresponding to the focal planes of the respective morphological images in same ranges as ranges of the respective morphological images.

3. The image processing device according to claim 1 or 2, wherein the first extraction means extracts the cell region from the morphological image by recognizing a cell nucleus.

4. The image processing device according to any one of claims I to 3, wherein the calculation means calculates the luminance value or a number of a fluorescent dye accumulated particle for each of the cell region extracted by the first extraction means.

5. The image processing device according to any one of claims 1 to 4, further comprising an image compositing means that generates one composite image by extracting, for each of the cell region extracted by the first extraction means, a partial image of a part corresponding to the cell region from the fluorescent image in the focal plane corresponding to the in-focus plane based on the coordinate and compositing all of the extracted partial image of each of the cell region.

6. An in-focus position specifying method comprising:
an input step that is inputting a morphological image and a plurality of fluorescent images, the morphological image representing a morphology of a cell in a tissue sample in which a biological substance is stained with a fluorescent nanoparticle, the biological substance being a single type of biological substance or a plurality of types of biological substances, and the plurality of fluorescent images having focal planes which are different at a predetermined interval in a height direction of the tissue sample in a same range as a range of the morphological image and representing expression of the biological substance in the tissue sample with a fluorescent bright spot;
a first extraction step that is extracting a cell region from the morphological image or a fluorescent image among the fluorescent images;
an in-focus plane specifying step that is specifying a focal plane which is most in focus as an in-focus plane for each of the cell region extracted by the first extraction step;
a coordinate specifying step that is specifying a coordinate in the in-focus plane of the cell region for each of the cell region extracted by the first extraction step;
a storage step that is storing the in-focus plane and the coordinate for each of the cell region extracted by the first extraction step;
a second extraction step that is extracting a fluorescent bright spot region from a fluorescent image among the fluorescent images which is in a focal plane, among the focal planes, corresponding to the in-focus plane for each of the cell region extracted by the first extraction step; and
a calculation step that is calculating a luminance value or a number of the fluorescent nanoparticle in the fluorescent bright spot region.

7. An in-focus position specifying program of a fluorescent image, the program causing a computer to function as:
an input means to input a morphological image and a plurality of fluorescent images, the morphological image representing a morphology of a cell in a tissue sample in which a biological substance is stained with a fluorescent nanoparticle, the biological substance being a single type of biological substance or a plurality of types of biological substances, and the plurality of fluorescent images having focal planes which are different at a predetermined interval in a height direction of the tissue sample in a same range as a range of the morphological image and representing expression of the biological substance in the tissue sample with a fluorescent bright spot;
a first extraction means that extracts a cell region from the morphological image or a fluorescent image among the fluorescent images;
an in-focus plane specifying means that specifies a focal plane which is most in focus as an in-focus plane for each of the cell region extracted by the first extraction means;
a coordinate specifying means that specifies a coordinate in the in-focus plane of the cell region for each of the cell region extracted by the first extraction means;
a storage means that stores the in-focus plane and the coordinate for each of the cell region extracted by the first extraction means;
a second extraction means that extracts a fluorescent bright spot region from a fluorescent image among the fluorescent images which is in a focal plane, among the focal planes, corresponding to the in-focus plane for each of the cell region extracted by the first extraction means; and
a calculation means that calculates a luminance value or a number of the fluorescent nanoparticle in the fluorescent bright spot region.
